Europäisches Patentamt

European Patent Office

Office européen des brevets

(19)

(11) Publication number: **0 184 914**
**A1**

(12) # EUROPEAN PATENT APPLICATION

(21) Application number: **85308368.1**

(22) Date of filing: **18.11.85**

(51) Int. Cl.⁴: **C 07 C 85/11, C 07 C 87/58**

---

(30) Priority: **05.12.84 US 678318**

(43) Date of publication of application: **18.06.86**
**Bulletin 86/25**

(84) Designated Contracting States: **AT BE CH DE FR GB IT LI LU NL SE**

(71) Applicant: **UNIROYAL CHEMICAL COMPANY, Inc., World Headquarters, Middlebury Connecticut 06749 (US)**

(72) Inventor: **Barrows, Franklin H., 230 Spring Street Naugatuck, New Haven Connecticut 06770 (US)**
Inventor: **Batorewicz, Wadim, 999 Whalley Avenue, New Haven Connecticut 06515 (US)**
Inventor: **Wheeler, Edward L., 126 Claxton Avenue Watertown, Litchfield Connecticut 06715 (US)**

(74) Representative: **Harrison, Michael Robert et al, Urquhart-Dykes & Lord 5th Floor Tower House Merrion Way, Leeds, LS2 8PA (GB)**

---

(54) **Process for preparing 4-aminodiphenylamines.**

(57) Uniformly high rates of hydrogenation in the noble metal catalyzed hydrogenation of a 4-nitrosodiphenylamine alkali metal salt in the presence of a water immiscible alcohol to the corresponding 4-aminodiphenylamine are achieved by the presence of added water during the hydrogenation.

D-6022

## PROCESS FOR PREPARING 4-AMINODIPHENYLAMINES

4-Aminodiphenylamines are widely used as intermediates in the manufacture of alkylated derivatives having utility as antiozonants, antioxidants and as stabilizers for gasolines. For example, reductive alkylation of 4-aminodiphenylamine (4-ADPA) with acetone provides the broadly used antiozonant N-isopropyl-N'-phenyl-4-phenylenediamine and reductive alkylation of 4-ADPA with methylethyl ketone as described in U.S. Patent No. 2,734,808 provides the gasoline stabilizer N-(sec-butyl)-N'-phenyl-4-phenylenediamine.

4-nitrosodiphenylamine (4-NDPA) is often prepared commercially from N-nitrosodiphenylamine (N-NDPA) using a variety of organic solvents or solvent mixtures. (The Chemistry of Amino, Nitroso and Nitro Compounds and Their Derivatives, Supplement F, p. 133, John Wiley & Sons (1982)). Due to the complexity of this reaction, a multiplicity of largely unidentified by-products is also generated, as shown by high pressure liquid chromatographic analysis of the reaction mixture. Some of these are severe poisons for noble metal catalysts used in the hydrogenation step. For this reason, 4-NDPA must be isolated from the reaction mixture by a variety of procedures prior to hydrogenation to 4-ADPA (see U.S. Patent Nos. 3,429,924, 3,748,362 and 4,034,042).

However, 4-NDPA is difficult to handle on a commercial scale. It is a deep blue or black powder having severe staining and dusting properties. It is ⸱ᴏ toxic and thermally unstable. To avoid the handling of solid 4-NDPA, it can be dissolved in an aqueous alkali metal hydroxide. The resulting solution of the alkali metal salt of 4-NDPA is then hydrogenated, usually over palladium on charcoal, to 4-ADPA as disclosed in U.S. Patent No. 2,974,169. In practice, this procedure is a purification by an extraction of 4-NDPA into the aqueous base away from the base insoluble by-products which remain dissolved in the organic solvent used in the preparation of 4-NDPA. The organic phase contains nitrosated species which are toxic and thermally unstable. Special costly procedures are, therefore, required for solvent recovery and safe disposal of the toxic residue. In addition, alkali metal salts of 4-NDPA in water solution slowly hydrolyze to aniline and 4-nitrosophenol resulting in a yield loss of a valuable intermediate.

The hazards of handling 4-NDPA in solid form have also been described in U.S. Patent No. 4,313,002. To overcome the problems of handling solid 4-NDPA, a process is disclosed therein where the 4-NDPA reaction mixture prepared by a Fischer-Hepp rearrangement in a mixture of inert organic solvents, usually an aromatic solvent and an aliphatic alcohol, is solubilized by the addition of an appropriate amount of an aqueous alkali metal hydroxide. The resulting organic phase contains the alkali metal salt of 4-NDPA in solution with all the water insoluble by-products. This solution is then hydrogenated over palladium on charcoal to 4-ADPA. The

-3-

0184914

hazardous nature of the by-products is rendered innocuous by the hydrogenation step so that the disposal of the process effluents is much less cumbersome.

The hydrogenation process described in U.S. Patent 4,313,002 has been found to be subject to significant limitations, however. The hydrogenation of alkali metal salts of 4-NDPA in the presence of the by-products of the rearrangement has been observed to be very erratic. This probably occurs as a consequence of some variability in the amount of catalyst poisons produced from run to run so that at a given catalyst loading, some batches can be hydrogenated rapidly and some not at all due to severe catalyst poisoning. Since it is not possible to determine beforehand the catalyst loading required for each batch to achieve a reasonably short uniform hydrogenation cycle from batch to batch, the process of U.S. Patent No. 4,313,002 is believed to have limited commercial usefulness.

## SUMMARY OF THE INVENTION

In accordance with the present invention, an improved process for converting a 4-nitrosodiphenylamine alkali metal salt to a 4-aminodiphenylamine is provided which comprises hydrogenating the 4-nitrosodiphenylamine alkali metal salt (which may be in the oxime form) dissolved in an essentially water-immiscible aliphatic saturated alcohol over a noble metal catalyst in the presence of an amount of added water sufficient to increase the rate of hydrogenation.

It has been discovered that addition of water to quantities of a 4-nitrosodiphenylamine alkali metal salt which previously required several hours for the conversion to the corresponding 4-aminodiphenylamine increases the hydrogenation rate to the point where the hydrogenation is completed in one hour or less. Conversely, addition of water to samples which do not exhibit inhibition of the noble metal catalyst does not accelerate the rate of hydrogenation. Thus, the addition of a hydrogenation rate-increasing amount of water to a solution of crude 4-nitrosodiphenylamine alkali metal salt prior to hydrogenation essentially eliminates unpredictably long hydrogenation cycles.

## DESCRIPTION OF THE PREFERRED EMBODIMENTS

While the improved process of the present invention is particularly useful for converting 4-NDPA alkali metal salt to 4-ADPA due to the commercial importance of the latter and will hereinafter be described primarily with reference to this compound, it is to be understood that the process is also applicable to the conversion of alkali metal salts of 4-nitrosodiphenylamines unsubstituted or substituted on one or more rings with $C_1-C_{12}$ alkyl. Examples of suitable substituted 4-nitrosodiphenylamines include 2'-alkyl, 4'-alkyl, 2,2'- and 2,4'dialkyldiphenylamines, where the alkyl group may be methyl, ethyl, propyl, butyl, pentyl, hexyl, decyl, and so forth.

The 4-NDPA alkali metal salt is obtained by contacting an aqueous solution of an alkali metal base with the 4-NDPA hydrochloride salt obtained from a Fischer-Hepp rearrangement of N-NDPA. For details of the rearrangement of N-NDPA to 4-NDPA, reference may be made to the disclosures of U.S. Patent Nos. 3,429,924, 3,728,392, 3,748,362, 4,034,042 and 4,479,008 among others. Examples of suitable alkali metal bases include sodium and potassium hydroxide. The concentration of the alkali metal base may be up to about 25% by weight, but normally it is in the range of from about 5 to about 20% by weight. The amount of base employed is equivalent to about 0.7 to about 1.5 molar proportions in excess of the amount of hydrogen halide used for rearrangement. For example, if 3 molar proportions of hydrogen halide is used in the rearrangement, then from about 3.7 to about 4.5 molar proportions of base is used. This is necessary to neutralize the excess acid, convert the salt of 4-NDPA to its free base and then convert the free base to its alkali metal salt.

The aliphatic saturated alcohol employed in this invention as reaction solvent is essentially immiscible with water. In the presence of 4-NDPA alkali metal salt, however, its miscibility with water is greatly enhanced so that a homogeneous solution is obtained. The saturated aliphatic alcohols useful as solvents for the present invention are primary or secondary, have a $C_5$ to $C_{10}$ hydrocarbon chain which can be linear or branched and have boiling points in the range of from about 130°C to about 200°C. The preferred alcohols of the present invention are primary alcohols having a $C_6$ to $C_8$

hydrocarbon chain. Examples of such alcohols are n-hexanol and 2-ethyl hexanol. A preferred alcohol is n-hexanol.

Hydrogenation of the 4-NDPA alkali metal salt to 4-ADPA can be conducted at pressures of from about 150 to about 250 psig. Higher $H_2$ pressure can be employed and is within the scope of this invention. However, pressure above 250 psig $H_2$ is not required since rapid consistent hydrogenation rates are achieved by adding water to the substrate. Hydrogenation temperatures range from about 40°C to about 80°C with from about 50°C to about 70°C being preferred. Any of the known and conventional noble metal hydrogenation catalysts, preferably a low commercially viable catalyst charge, serve to effectuate the desired reduction. Examples of suitable catalysts include palladium and platinum. Such catalysts can be used either unsupported or supported. If supported, they may be used on such supports as charcoal, keiselguhr, alumina, silica and the like with charcoal being preferred. The preferred catalyst of this invention is Pd supported on charcoal in concentrations ranging from about 1% to about 10% Pd by weight preferably 5% Pd by weight. The noble metal catalyst can be used in amounts of from about 0.01 grams to about 0.025 grams, preferably from about 0.015 to about 0.020 grams, per mole of 4-NDPA alkali metal salt. Obviously, larger amounts of catalyst can be employed in the reactions of this invention however, this is usually commercially undesirable.

The amount of water required to overcome the intermittent inhibition of hydrogenation can vary widely and is generally from about 100 grams to about 300 grams per mole of 4-NDPA salt. Preferably, from about 150 to about 250 grams of water per mole is employed to insure consistent fast reduction rates. Higher amounts of water, although not detrimental to the reduction rates, are not required and may in fact cause the onset of hydrolysis of the 4-NDPA salt to aniline and 4-nitrosophenol.

## EXAMPLE 1

This example illustrates the preparation of 4-ADPA starting with diphenylamine (DPA).

A.   Nitrosation of DPA to N-NDPA

To a 2-liter resin flask, fitted with a mechanical stirrer, a thermometer, and an addition funnel, was charge n-hexanol (411g), DPA (169g., 1.0 mole) and 40% $NaNO_2$ solution (71.7g of 99% $NaNO_2$ dissolved in 108g of water). A 35% sulfuric acid solution (59.6g of 96% $H_2SO_4$ dissolved in 111g of water) was added dropwise to the stirred reaction mixture over a period of 30 minutes, allowing the temperature to rise to 45°C toward the end of the acid addition. The mixture was then transferred to a 2-liter separatory funnel and while still hot, the water phase was separated and discarded. It is necessary to keep the phases hot during the separation because N-NDPA at 34% concentration tends to crystallize out at lower temperatures.

B.    Rearrangement to 4-NDPA.HCl

The N-NDPA-hexanol solution was returned to a clean 2-liter resin flask fitted with a mechanical stirrer, a baffle, a thermometer and a gas inlet tube. HCl gas (80.4g, 2.2 moles) was added subsurface through the gas inlet tube from a lecture bottle resting on an electronic balance. The rate of HCl addition was adjusted using the balance and a flowmeter so that the addition of the gas could be completed in 3.8 hours. The temperature profile of the rearrangement was as follows: for the first 2 hours the temperature was held at 32°C, then it was gradually raised to 40°C over a period of 1.8 hours and subsequently held at 40°C for 0.5 hours. The agitation of the mixture throughout the rearrangement was set at about 200 rpm.

C.    Conversion to 4-NDPA Sodium Salt

A 17% NaOH solution (176g of 50% NaOH, 2.2 moles, dissolved in 344g of water) was added dropwise to the stirred reaction mixture over a period of 30 minutes keeping the temperature between 30-40°C by means of an ice-water bath. The NaOH addition was stopped when a pH of 7-8 was reached, as shown by a pH meter. Then 80g of 50% NaOH solution (1.0 mole) was added rapidly. The phases were allowed to settle and the aqueous layer was separated and discarded. For the split batch studies, the hexanol solution was divided in half. One half was hydrogenated with water added and the other without.

D.    Hydrogenation of 4-NDPA Sodium Salt to 4-ADPA

One half of the 4-NDPA sodium salt reaction solution

from step C was charged in a 1-liter stirred autoclave with 0.15 (or 0.20) grams of 5% Pd on charcoal and hydrogenated under 150 to 250 psig $H_2$ pressure at 60°C until $H_2$ absorption ceased. The resulting two phase mixture was filtered to remove the catalyst and the lower aqueous layer discarded. The organic phase was washed with water (100g portions) until the washings were neutral. Hexanol was removed by distillation and the residue subjected to a vacuum distillation under 1-2 mm Hg to isolate 4-ADPA from the non-volatile by-products. The distilled 4-ADPA was analyzed by gas chromatography to determine the purity of the product which ranged from 97 to 98%.

### EXAMPLE 2

The unexpected beneficial effect of the added water on the hydrogenation rates was demonstrated in the following manner. A series of crude 4-NDPA salt batches was prepared as described in Example 1. One half of each batch was hydrogenated without added water. If the reduction took longer than one hour, water was added to the other half and hydrogenation was repeated. In this manner, the effectiveness of the water could be shown in an unambiguous fashion since the hydrogenation was performed on an identical pair of substrates. This was crucial because of the extreme variability of hydrogenation rates from batch to batch.

In the following table, the results of the hydrogenation of the identical pairs with and without the added water are summarized. The data clearly show the profound

0184914

effect of the added water on the hydrogenation rates for the batches that showed slow hydrogenation rates, i.e., Runs II, III and IV.

Run I illustrates the case where no inhibition of the hydrogenation was seen and, consequently, added water had no effect on the rate.

-11-

0184914

## TABLE

<u>HYDROGENATION OF CRUDE 4-NDPA SODIUM SALT IN HEXANOL</u>

| RUN I | | 5% Pd/C g./mole | H₂O added g./mole | Reduction Hrs.(1) | % 4-ADPA(2) |
|---|---|---|---|---|---|
| | A | 0.4 | None | 0.3 | 92.5 |
| | B | 0.4 | 200 | 0.3 | 92.0 |
| RUN II | | | | | |
| | A | 0.4 | None | 12.6 | (not isolated) |
| | B | 0.4 | 240 | 0.7 | 91.4 |
| RUN III | | | | | |
| | A | 0.3 | None | 3.2 | 89.6 |
| | B | 0.3 | 240 | 1.0 | 89.4 |
| RUN IV | | | | | |
| | A | 0.3 | None | Very Slow | - |
| | B | 0.3 | 120 | 0.5 | 90.7 |

(1)   60°C under 150-250 psig $H_2$ pressure

(2)   Separated by distillation, 97-98% 4-ADPA assay by GC.

. CLAIMS:

1. A process for preparing a 4-aminodiphenylamine characterised in that it comprises hydrogenating a 4-nitrosodiphenylamine alkali metal salt unsubstituted or substituted on one or more rings with $C_1-C_{12}$ alkyl groups in the presence of an essentially water-immiscible aliphatic saturated alcohol and a hydrogenation rate-increasing amount of water over a noble metal catalyst.

2. A process according to claim 1 characterised in that the alcohol is a primary or secondary linear or branched alcohol having from 5 to about 10 carbon atoms.

3. A process according to claim 1 or claim 2 characterised in that the alcohol has from 6 to 8 carbon atoms and has a boiling point in the range of from about 130°C to about 200°C.

4. A process according to any of the preceding claims characterised in that the alcohol is n-hexanol.

5. A process according to any of the preceding claims characterised in that from about 100 to about 300 grams of water is added per mole of 4-nitrosodiphenylamine alkali metal salt.

6. A process according to any of the preceding claims characterised in that from about 150 to about 250 grams of water is added per mole of 4-nitrosodiphenylamine alkali metal salt.

7. A process according to any of the preceding claims characterised in that the noble metal is palladium.

8. A process according to any of the preceding claims characterised in that the noble metal is palladium on a charcoal support.

9. A process according to any of the preceding claims characterised in that the 4-nitrosodiphenylamine alkali metal salt is a 4-nitrosodiphenylamine sodium salt.

10. A process according to any of the preceding claims characterised in that either or both phenyl rings of the 4-nitrosodiphenylamine alkali metal salt are substituted with one or more $C_1$-$C_{12}$ groups.

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | GB-A- 947 082 (ICI)<br>* Claims * | 1 | C 07 C 85/11<br>C 07 C 87/58 |
| Y | FR-A-2 019 340 (ICI)<br>* Claims * | 1 | |
| D,Y | US-A-4 313 002 (T. SIMON et al.)<br>* Claims * | 1 | |

---

**TECHNICAL FIELDS SEARCHED (Int. Cl.4)**

C 07 C 85/00
C 07 C 87/00

The present search report has been drawn up for all claims

| Place of search | Date of completion of the search | Examiner |
|---|---|---|
| THE HAGUE | 12-03-1986 | MOREAU J.M. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503 03 82